# EUROPEAN PATENT SPECIFICATION

(11) **EP 2 679 584 B1**
(45) Date of publication and mention of the grant of the patent: **03.08.2016**
(21) Application number: 12750125.2
(22) Date of filing: 20.02.2012
(51) Int. Cl.: C07D 345/00, A61K 31/095, A61K 47/40, A61K 47/36, C08B 37/00, A61P 39/00

(54) **CLATHRATE COMPLEX OF CYCLODEXTRIN OR ARABINOGALACTAN WITH 9-PHENYL-SYM-OCTAHYDROSELENOXANTHENE**
CLATHRATKOMPLEX AUS CYCLODEXTRIN ODER ARABINOGALACTAN MIT 9-PHENYL-SYM-OCTAHYDROSELENOXANTHEN
COMPLEXE DE CLATHRATES DE CYCLODEXTRINE OU D'ARABINOGALAXANE ET DE 9-PHÉNYL-SIMM-OCTAHYDROSÉLÉNOXATHÈNE

(30) Priority: 21.02.2011 RU 2011106350
(43) Date of publication of application: 01.01.2014
(73) Proprietor: L-PDSK Limited company, Obninsk, Kaluga region, 249030 (RU)
(72) Inventor: TSYB, Anatoliy Fedorovich, Obninsk Kaluzhskaya obl. 249030 (RU); GONCHAROVA, Anna Yakovlevna, Obninsk Kaluzhskaya obl. 249034 (RU); ROZIEV, Rahimdzhan Ahmetdzhanovich, Obninsk Kaluzhska a obl. 249030 (RU); VOROBYEV, Ilya Vladimirovich, Obninsk Kaluzhskaya obl. 249034 (RU); PODGORODNICHENKO, Vladimir Konstantinovich, Obninsk Kaluzhskaya obl. 249032 (RU); ERIMBETOV, Kenes Tagayevich, pos. Instituta Borovsk Kaluzhskaya obl. 249013 (RU)
(74) Representative: Anohins, Vladimirs
(86) International application number: PCT/RU2012/000117
(87) International publication number: WO 2012/115538

(56) References cited:
- EP-A1- 1 348 432
- CN-A- 1 853 627
- RU-C1- 2 317 074
- RU-C1- 2 374 238
- E. V. RATUSHNAYA: PHARMACEUTICAL CHEMISTRY JOURNAL, vol. 36, no. 12, 1 January 2002 (2002-01-01), pages 652-653, XP055133390, ISSN: 0091-150X, DOI: 10.1023/A:1023405611793

## Description

The invention relates to novel clathrate complexes of cyclodextrin (hereinafter CD) or arabinogalactan (hereinafter AG) with 9-phenyl-sym-octahydroselenoxanthene, which can be used in the pharmaceutical industry, and methods for the production thereof. The invention also relates to compositions and medicinal agents based on novel clathrate complexes of cyclodextrin and arabinogalactan with 9-phenyl-sym-octahydroselenoxanthene. The complexes may be obtained with the use of α-, β-, or γ-cyclodextrins, hydroxypropyl-β-cyclodextrin, Siberian larch arabinogalactan.

Addressing drug transport problem, clathrate complex preparation has a significant impact on almost every route of drug administration: from oral to injectable. Due to physical and chemical profiles, CD and AG clathrate complexes make it possible to increase the absorption of therapeutic agents that are characterized by low bioavailability, and, consequently, to reduce the dosage of the applied drug (for example refer to Groman E.V., Enriquez P.M., Jung Chu, Josephson L. Arabinogalactan for hepatic drug delivery, Bioconjugate Chem. 1994. No.5, pp. 547-556; Medvedeva S.A., Alexandrova G.P., Dubrovina V.I., Chetverikova T.D., Grischenko L.A., Krasnikova I.M., Feoktistova L.P., Tyukavkina N.A.) (in Butlerov Commun. 2002. No.7, pp. 45-49) describe the prospects of AG as a polymer matrix for biogenous metals). New forms and routes of drug transport may expand the therapeutic potential of the prescribed treatment, allowing the delivery of new kinds of drugs to previously inaccessible areas of the human organism.

Cyclodextrins and arabinogalactan used for preparing are well-known complexing agents. Cyclodextrin molecules have a toroidal shape with a hydrophobic internal cavity. Soluble intermolecular complexes of lipophilic organic compounds are formed in the solution due to the intercalation of molecules thereof in CD cavity. Arabinogalactan is a polysaccharidic metabolite of endemic Siberian forest flora, namely Siberian larch (*Larix sibirica*) and Gmelin larch (*Larix gmelinii*) and can effortlessly be extracted from the wood of these species. Chemically AG is a polysaccharide with a comb-shaped structure and molecular mass of 9-18 kD. The backbone is identified to consist of galactose units. The side chains are composed of arabinose and galactose monomers. The branched structure of AG and the presence of many terminal galactose and arabinose groups facilitate the formation of strong intermolecular complexes with therapeutic agents, molecules of which most likely bind through the intermolecular hydrogen bonds in the space between the side chains. This space may vary because of the conformational mobility of arabinogalactan macromolecules, thus contributing to the formation of intermolecular clathrate complexes with a wide range of substances. The model experiments showed that larch AG possesses high membrane affinity (Kolhir V.K., Tyukavkina N.A., Baginskaya A.I., Mineeva M.F. et al., Evaluating the Pharmacological Properties of Arabinogalactan // Abstracts of the III Russian National Congress "Man and Medicine", Moscow, 1996, p. 27). It is known that the ability of some substances to penetrate selectively the membrane barriers is by virtue of the presence of galactose units in their structure (Gryshchenko L.A. Hydrodynamic and Molecular-Weight Characteristics of Iron-derived Arabinogalactan. // Abstracts of the Youth Scientific Conference on Organic Chemistry "Baikal Readings - 2000". Irkutsk, 2000, p. 26). It was shown that the AG from western larch can serve as a carrier for targeted delivery of diagnostic and therapeutic agents, as well as enzymes, nucleic acids, vitamins or hormones that are transported to specific cells, in particular hepatocytes (parenchymal liver cells). A complex is formed between the delivered agent and arabinogalactan, which is able to interact with the asialoglycoprotein receptors of cells (see, for example, Groman E.V., Menz E.T., Enriquez P.M., Jung C. Delivery of Therapeutic Agents to Receptors using Polysaccharides, Patent US5544386. 1996 // CA 1996, v. 125, p. 284 915z). The Public Chemical Journal "Butlerov Communications" (2002, No. 7, pp. 45 - 49) discloses AG as a promising polymer matrix for biogenic metals.

The prospects for the development of targeted delivery systems and transport mechanism through the mucosa is described in the application of RU2005115883, published in 2006. The overall potential of drug transport systems based on cyclodextrin inclusion complexes was assessed in Russian patent RU2121830, issued in 1998. In addition to pharmaceutical industry, complexes of active substances with cyclodextrins and arabinogalactan have also been applied in cosmetology. For instance, in demand are complexes of phyto amino acids included in the cyclodextrin capsules. Similar complexes of cyclodextrin with monosaccharides or uronic acids are known.

The patent RU2121830 (published in 1998) discloses water-soluble pharmaceutical composition and method for preparing thereof for such well-known drugs as Sibazon, Azaleptin, Mezapam, Indomethacin. Pharmacological trials of described complexes in laboratory animals showed a several-fold reduction of therapeutic doses of these drugs.

There are complexes of β-cyclodextrin with nonsteroidal anti-inflammatory drugs (paracetamol, ibuprofen, ketoprofen, flufenamic and mefenamic acids, etc.), steroids, prostaglandins and prostacyclins, barbiturates, sulfonamides, cardiac glycosides and other drugs (see, for example, Patents US4524068, US4727064, Patent RU2337710, issued in 2008, J. Szejtli, Industrial Applications of Cyclodextrins // In Inclusion Compounds, v.3. ed. Atwood J.L., Davies J.E., Mcnicob D.D., Academic Press, NY., 1984, pp .331 - 390).

A number of publications describe the inclusion compounds (clathrate complexes) of α-or β-cyclodextrin with antiviral compounds, preparation methods and use thereof (see, for example, the application RU 2005114097, Patents RU2288921, RU2377243, RU2247576, DE19814815A1, DE19814814A1, US4956351, US5221669, applications US 2005/0209189, US 2005/0281872, US 2009/0286757, JP10-045319, JP58-092691, JP-2005-179329, JP-9-015632).

In Patent US5478576 (1995) "Arabinogalactan derivatives and uses thereof", the authors (Jung Chu, Enriquez P.M., Palmacci P., Josephson L., and Lewis J.M.) disclose conjugates of AG with adenosine monophosphate (AMP) or adenine-9-β-D-arabinofuranoside-5'-monophosphate, which have antiviral activity. In another embodiment is described the conjugates of AG with S-2-(3-aminopropylamino)ethylthiophosphoric acid having radioprotective properties. In "Synthesis and Characterization of Novel Watersoluble Amphotericin B-Arabinogalactan Conjugates" (Biomaterials, 2002, V. 23, N5, p. 1327-1335) Ehrenfreund-Kleinman T., Azzam T., Falk R., Golenser J., and Domb A.J. describe a water-soluble complex of AG with an insoluble antibiotic amphotericin B used for treatment of fungal diseases. Utilization of such a complex facilitates its application, significantly decreases the toxicity and enhances the drug efficacy.

The Patent RU2278669 reveals a liquid-phase method for obtaining arabinogalactan-couples water-soluble silver compound, representing water-soluble particles of 10-30 nm. Another Patent RU2337710 discloses a solid-phase method for producing a water-soluble pharmaceutical formulation with enhanced pharmacological activity, consisting of slightly water-soluble drug substances, such as Sibazon, Indomethacin, Mezapam, Azaleptin. As a result, the drug substances form water-soluble intermolecular complexes with AG in a mass ratio of from 1:5 to 1:20. The Patent RU2128664, published on 10.04.1999, describes the inclusion compounds of 9-(2-oxyethoxy)methylguanine (acyclovir) with β-cyclodextrin, as having selective activity against herpes simplex virus, as well as liquid- and solid-phase methods for preparation thereof.

The liquid-phase method consists of gradual dissolution of the components at a specific ratio in water or aqueous-alcohol solution under heating, followed by concentration of the resulting product and isolation of the final product. Solid-phase method involves the mechanical grinding of crystalline acyclovir and β-cyclodextrin mixture in a vibratory mill. In these circumstances, the product remains in the form of fine crystalline fluidized powder. The patents RU2242974, RU2357968, RU2377243 describe crystalline forms and clathrate nanoforms of cyclodextrin and anti-inflammatory drugs. According to Patent RU2377243, the solid-phase method involves the preparation of solid dispersions of the components, followed by optional milling or grinding of the dispersion.

The patents RU2213092, RU2239632, RU2281007 describe processes for preparing substituted sym-octahydroselenoxanthenes, including 9-phenyl-sym-octahydroselenoxanthene (also known as selenopyran), which can be used in medicine, pharmacology, food and cosmetic industries. Meralenko O.V. (from Belgorod Agricultural Academy) pointed to the high toxicity of 9-phenyl-sym-octahydroselenoxanthene. The Patent RU2374238 describes alpha-crystalline form of the compound having low toxicity, as well as antioxidant, detoxifying, immunomodulatory, antiatherogenic, hypolipidemic and anabolic activity. The patent RU2317074 shows that 9-phenyl-sym-octahydroselenoxanthene is an inhibitor of differentiation of hematopoietic progenitor cells and can be used to improve the efficiency of irradiation and cytotoxic drugs by reducing the damaging effects of the mentioned agents on the process of hematopoiesis, thereby providing more effective protection of normal tissues.

The goal of the present invention is to survey new clathrate complexes of 9-phenyl-sym-octahydroselenoxanthene with cyclodextrins or arabinogalactan. The clathrate complex of 9-phenyl-sym-octahydroselenoxanthene is characterized by increased water solubility, improved bioavailability, reduced dose that will ultimately lead to decreasing the toxicity of the mentioned compound. Another task of the present invention consists in the development of new preparation approaches of the mentioned clathrate complexes and their application in pharmaceutical compositions and medicaments.

According to the this invention, the proposed novel compounds are clathrate complex (or inclusion compound) of 9-phenyl-sym-octahydroselenoxanthene of Formula I with α-, β-, or γ-cyclodextrins, hydroxypropyl-β-cyclodextrin, or larch arabinogalactan, preferably Siberian larch AG, at a molar ratio of from 1:1 to 1:10 (mass ratio of 1:3 to 1:30) in the case of α-, β- or γ-cyclodextrin or hydroxypropyl-β-cyclodextrin and at a mass ratio of from 1:10 to 1:20 in case of arabinogalactan.

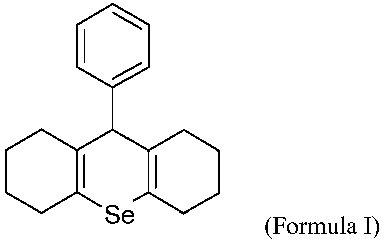

Preferable clathrate complexes are complexes with β-cyclodextrin with a molar ratio of 9-phenyl-sym-octahydroselenoxanthene:β-cyclodextrin as 1:3 or 1:5 (mass ratio 1:10 or 1:17), and mass ratio of 9-phenyl-sym-octahydroselenoxanthene:AG equal to 1:10 or 1:15. These ratios allow to completely transfer of 9-phenyl-sym-octahydroselenoxanthene into the clathrate complex, significantly influencing the water-solubility and bioavailability of the compound. The water-solubility of 9-phenyl-sym-octahydroselenoxanthene is almost zero, whereas the solubility of its complex with β-cyclodextrin at a mass ratio of 1:10 is 0.443 g/l, while at the mass ratio 1:17 is 0.395 g/l. For arabinogalactan these values are 0.480 and 0.362 g/l for complexes 1:10 and 1:15, respectively.

The invention also relates to pharmaceutical compositions containing the above-mentioned clathrate complex of α-, β- or γ-cyclodextrin, hydroxypropyl-β-cyclodextrin or arabinogalactan coupled with 9-phenyl-sym-octahydroselenoxanthene at the above-mentioned molar ratios, perhaps in the form of nanoparticles with a size less than 100 nm, in an effective quantity and a pharmaceutically acceptable carrier and/or excipient.

The invention also relates to a medicament in the form of capsules and tablets in pharmaceutically acceptable packing, comprising a clathrate complex of α-, β- or γ-cyclodextrin, hydroxypropyl-β-cyclodextrin or AG with 9-phenyl-sym-octahydroselenoxanthene at the aforementioned molar ratio, or the pharmaceutical compositions based on the said complex in an effective amount.

Moreover, the clathrate complexes proved to be useful for the majority of dosage forms and administration routes.

The term "effective amount" and "effective quantity", as used herein, implies such an amount of the compound with formula 1, the use of which, together with its toxicity and activity indices and based on the available knowledge of a skilled in the art, should be effective for a given pharmaceutical composition or dosage form.

If necessary, the pharmaceutical composition may contain auxiliary agents such as fillers, humectants, emulsifiers, suspending agents, thickeners, sweeteners, flavoring agents, fragrances. Pharmaceutically acceptable additives may be selected from, for example, microcellulose, lactose, calcium stearate, starch. The selection and ratio of these components depends on the nature, route of administration and dosage.

The content of the active ingredient is usually from 1 to 20 wt%, in combination with one or more pharmaceutically acceptable additives, such as diluents, binders, disintegrants, adsorbents, fragrances, flavoring agents.

The mentioned pharmaceutical composition and medicament may be prepared by known pharmaceutical methods.

For preparing the pharmaceutical composition, the active ingredient (compound of formula 1) is mixed with a pharmaceutically acceptable carrier and, if necessary, with appropriate additives (carriers and/or excipients), for example, to improve the taste, odor.

The medicament may be in liquid or solid form.

Examples of solid dosage forms are tablets, pills, gelatinous capsule, etc. Examples of liquid dosage forms for injections and parenteral administration are solutions, emulsions, suspensions. Preparation of these dosage forms is possible by employment of conventional pharmaceutical methods: by mixing the components, tableting, encapsulation, etc.

The proposed herein clathrate complexes may be prepared in two ways:
1) through a liquid-phase synthesis method;
2) through a solid-phase synthesis method.

The liquid-phase method includes the preparation of initial aqueous solutions of cyclodextrin or arabinogalactan and 9-phenyl-sym-octahydroselenoxanthene in an organic solvent miscible with water (for instance, acetone). These solutions are later mixed by agitation and heating till up to 70°C, at a molar ratio of 9-phenyl-sym-octahydroselenoxanthene:cyclodextrin of from 1:1 to 1:10 (mass ratio from 1:3 to 1:30) or at a mass ratio of 9-phenyl-sym-octahydroselenoxanthene:arabinogalactan of from 1:10 to 1:20, with subsequent stirring at indicated temperature until a homogeneous solution is obtained. The resulting crystalline clathrate complex is isolated and, if necessary, is further grounded at conditions mentioned below for the formation of nanoparticles of the resulting product with a size less than 100 nm. For the complex 9-phenyl-sym-octahydroselenoxanthene: β-cyclodextrin the molar ratios of 1:3 or 1:5 (mass ratios of 1:10 and 1:17) are preferable, while for the complex 9-phenyl-sym-octahydroselenoxanthene:arabinogalactan - 1:10 or 1:15 (by weight).

As the solvent miscible with water acetone, ethanol, isopropanol, 1,4-dioxane, and any other water-miscible solvent can be used.

The approach described herein generates non-covalent clathrate complexes stabilized by hydrogen bonds. Non-covalent complex is a complex that is formed between the molecules of substances in a suitable solvent due to intermolecular non-covalent van der Waals attraction, namely, hydrogen bonding and coordinated-ionic bond forming.

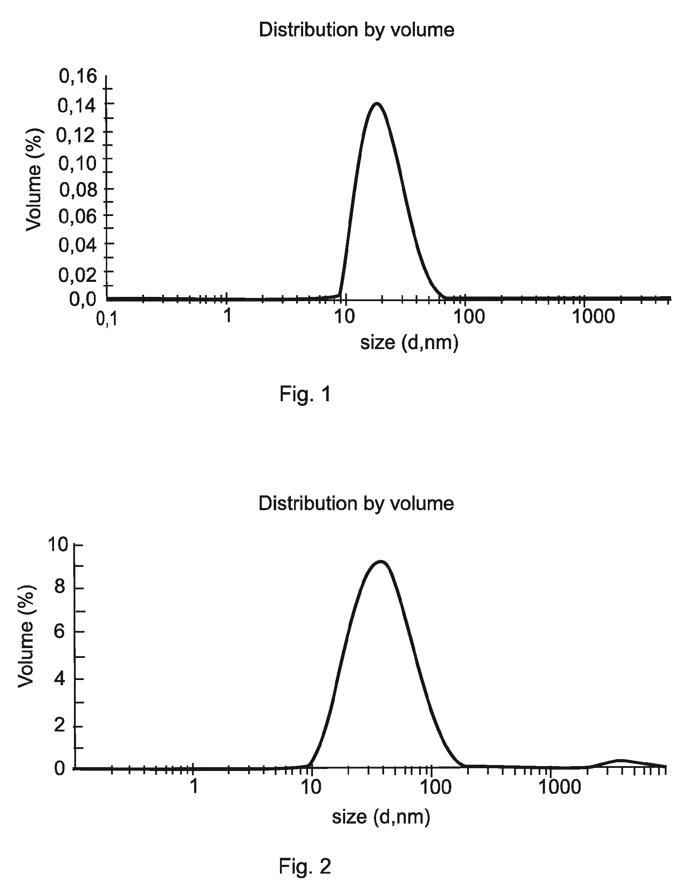

The solid-phase method is characterized by the fact that corresponding cyclodextrin or arabinogalactan and 9-phenyl-sym-octahydroselenoxanthene are grinded at a speed from 100 rpm until 1000 rpm during 10 to 60 minutes at a molar ratio of 9-phenyl-sym-octahydroselenoxanthene:cyclodextrin from 1:1 to 1:10 (mass ratio from 1:3 to 1:30) or mass ratio of 9-phenyl-sym-octahydroselenoxanthene:arabinogalactan from 1:10 to 1:20 at 20 to 50°C. Where required, the obtained inclusion compounds (clathrate complexes) can be isolated in the form of nanoparticles with a size up to 100 nm, preferably close to 24.3 nm for the complex 9-phenyl-sym-octahydroselenoxanthene:cyclodextrin (Fig. 1) and 32.5 nm for the 9-phenyl-sym-octahydroselenoxanthene:arabinogalactan (Fig. 2). Production of the clathrate complex in the form of nanoparticles with a specified size is confirmed by the analysis carried out on *Zetasizer Nano ZS.*

The theory of mechanical synthesis suggests that plastic deformation of solids usually leads not only to alteration of the solid shape, but also to the accumulation of defects that change the physical and chemical properties, including reactivity. Accumulation of defects can be used in chemistry for enhancing the rate of chemical reactions involving solids, reducing the reaction temperature, as well as for other ways of chemical reactions intensification in the solid phase.

As a result of mechanical treatment, a unique feature of solids activation process is that it happens during grinding when the particle size reaches a certain critical value. During mechanical activation, defects accumulation throughout the crystal occurs at a bigger extent than the increase of the surface. This dramatically changes many physical and chemical properties of solids, including reactivity.

Increased reactivity due to mechanical activation can be considered as a method of obtaining solids in a metastable, active form. Since the chemical reactions involving solids depend on the characteristics of their mechanism and possess different sensitivity to various **crystal** defects, the aim of mechanical activation consists not only in the defects accumulation, but also in receiving a particular type of defects that is required for a reaction. This goal can be achieved through both: selection of the conditions for mechanical impact on crystal (impact energy, duration, pressure and shear relationship, treatment temperature, composition of the ambient atmosphere), and consideration of the crystal's structural features, nature of the chemical bond, its strength characteristics, etc.

Production of nanoparticles usually starts with solid dispersion accumulation followed by milling or grinding of the solid dispersion until the appropriate particle size is obtained. Fine grinding of the particles can be achieved by employment of mechanical forces. Such a force may be attained by the collision of particles at a high rate. For example, production of finely powdered particles can be accomplished by fine grinding using an air-jet micronizer, a ball mill or a pin mill. The size of received nanoparticles can be determined by any conventional approach known in the art. The following methods might be applied:
sieving, sedimentation, electrical sensing (by Coulter counter), microscopy, Low-Angle Laser Light-Scattering (LALLS).

The invention discloses additional details in the following examples, which, however, do not limit the claims of the applicant, merely illustrating the possibility of its implementation. Unexpected and unobvious for the clathrate complexes, the described herein compounds possess enhanced therapeutic effects as compared to other compounds with similar structure that are widely used in clinical practice.

### The following examples illustrate the invention:

1. Liquid-phase method. The weighted amount 50 g (0.045 mole) of β-cyclodextrin was dissolved in 0.3 1 of distilled water at 55-60°C. The weighted amount 5 g (0.015 mole) of 9-phenyl-sym-octahydroselenoxanthene was dissolved in 0.7 1 of acetone at 55°C. Under stirring and heating the solution of 9-phenyl-sym-octahydroselenoxanthene, was added to the β-cyclodextrin solution. After mixing, the temperature was maintained at 55-60°C for a real solution formation, being afterwards decreased to 45 °C during 2 hours. The acetone and some water evaporated from the solution while the residue was lyophilized. As a result, a complex of 1:3 (molar ratio) and 1:10 (mass ratio) is obtained. The complex is in form of white crystalline powder with a total weight of 55 g. The resulting complex is further milled as described above.
   Similarly, the clathrate complex of 9-phenyl-sym-octahydroselenoxanthene and AG was received. 5 g of 9-phenyl-sym-octahydroselenoxanthene and 50 g of arabinogalactan were used. As a result, a light yellow crystalline powder, representing the clathrate complex (mass ratio 1:10), was obtained.
2. Solid-phase method. The tests were conducted on a ball planetary mill *Aktivator 2S* with the grinding jars made from corundum and balls made from zirconium oxide with 3, 5 and 10 mm diameter at velocity between 100 rpm and 1000 rpm. Optimal regimes were 500 rpm for 5 min, 700 rpm for 10 min, 500 rpm for 10 min, as well as 600 rpm for 5 min, 800 rpm for 10 min and 600 rpm for 10 min. The molar ratio varied for 9-phenyl-symoctahydroselenoxanthene:β-cyclodextrin from 1:1 to 1:10 (mass ratio from 1:3 to 1:30, respectively). The optimal molar ratio turned to be 1:3 or 1:5 (mass ratio 1:10 or 1:17). The resulting clathrate complex is isolated and, if necessary, is further milled as described above. Clathrate complex is obtained in form of fine white fluidized powder (the average particle size in the case of β-cyclodextrin is 24.3 nm (Fig. 1). Under similar conditions, the clathrate complexes of 9-phenyl-sym-octahydroselenoxanthene and AG is obtained. Their optimal mass ratios is of 1:10 or 1: 15. The complex is identified as a fine fluidized light yellow powder with an average particle size of 32.5 nm (Fig. 2). All complexes obtained had optimum set of spectral data and solubility.

Similarly, the following complexes have been obtained:
9-phenyl-sym-octahydroselenoxanthene:α-cyclodextrin (molar ratio of 1:1, 1:3, 1:5, 1:10);
9-phenyl-sym-octahydroselenoxanthene:γ-cyclodextrin (molar ratio of 1:1, 1:3, 1:5, 1:10);
9-phenyl-sym-octahydroselenoxanthene:hydroxypropyl-β-cyclodextrin (molar ratio of 1:1 1:3, 1:5, 1:10);
9-phenyl-sym-octahydroselenoxanthene:arabinogalactan (mass ratio of 1:10, 1:15, 1:20).

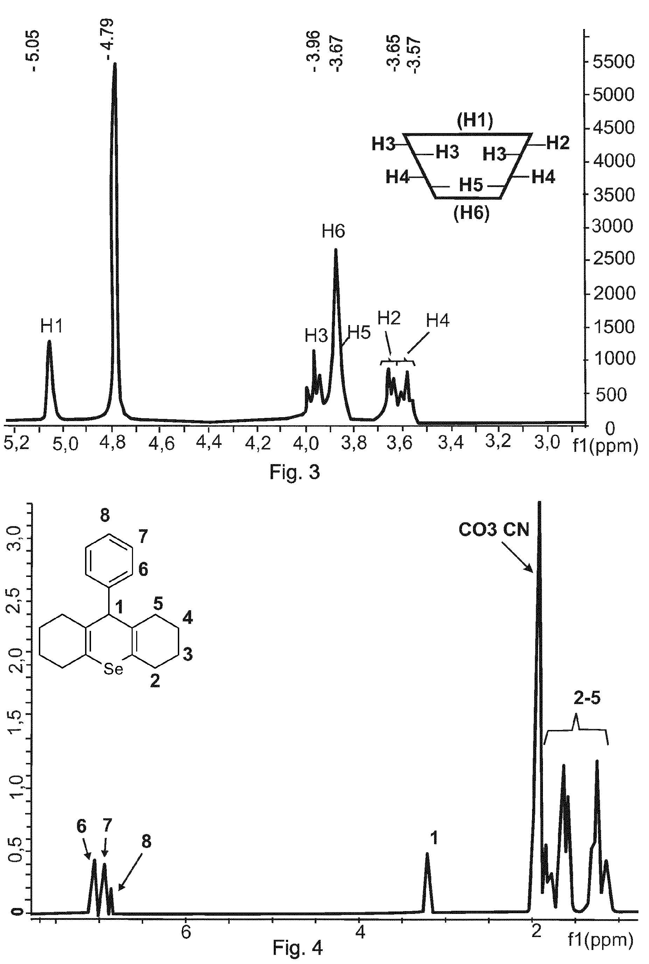

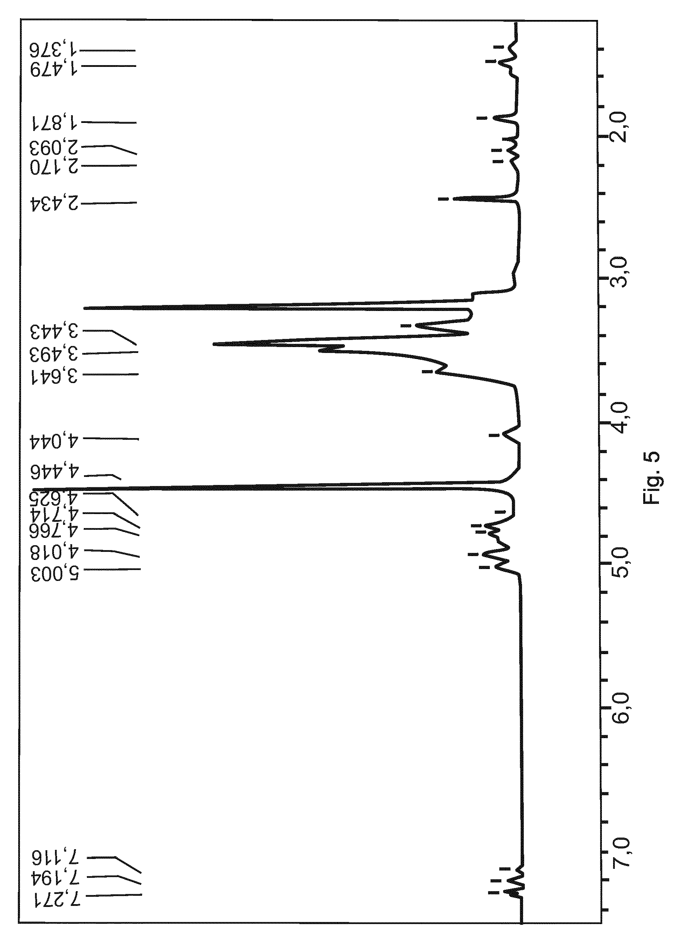

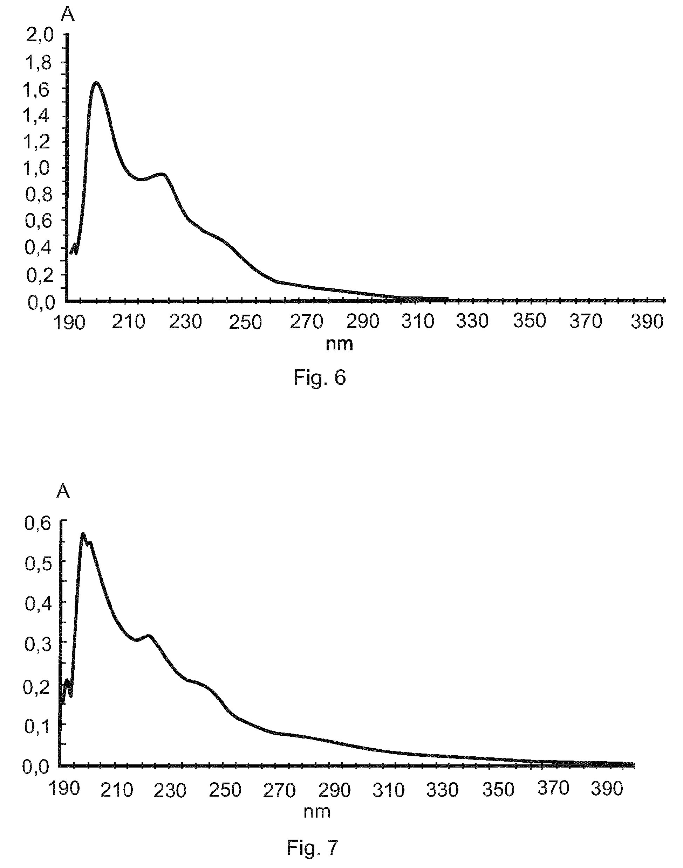

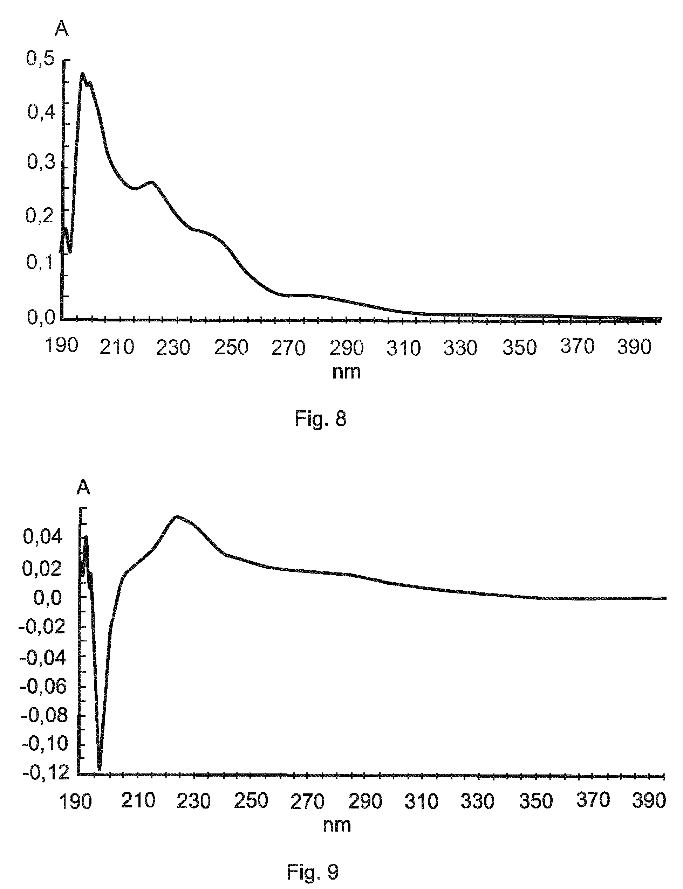

The formation of the complexes was confirmed by proton magnetic resonance (PMR) spectroscopy, UV and IR spectroscopy. Moreover, data on improved water-solubility of the complex compared to the initial substances was obtained.
1. The complex of 9-phenyl-sym-octahydroselenoxanthene (hereinafter **compound 1, selenox**):β-cyclodextrin (hereinafter **compound 2**), in a molar ratio of 1:3, was subjected to comparative analysis of PMR spectra of β-cyclodextrin, Compound 1 (selenox) and Compound 2 (the complex) (see **Figs. 3, 4** **and** **5**, respectively). The analysis showed that the complex spectrum contained signals different from those of β-cyclodextrin and selenox. Since the spectrum was taken in deuterated water (selenox is not soluble in water), one may consider that an inclusion complex, not a mechanical mixture, was formed. It should also be noted that the β-cyclodextrin signals appear double, suggesting that, apparently, at least two cyclodextrin molecules are involved in selenox inclusion.
2. Comparative UV spectra of Compound 1, Compound 2 (at a ratio of 1:3 and 1:5), as well as of pure β-cyclodextrin and selenox were obtained. The results are shown in **Figs. 6, 7****,** **8 and 9**, respectively. Changes in the indicators intensity could be seen at the angle 2θ.

**Solvent: ethanol:water = 3:2. Dissolution carried out by heating**

| | |
|---|---|
| Compound **1** | 0.01 g in 50 ml |
| CD: Compound **2** = 3:1 | 0.11 g in 50 ml of solvent |
| CD: Compound **2** = 5:1 | 0.18 g in 25 ml of solvent |
| CD (cyclodextrin) | 0.17 g in 50 ml of solvent |

To obtain the spectra these solutions were diluted 20 times (0.2 ml + 4 ml of solvent).

The weighted amount of the samples contained the same amount of Compound 1 (0.01 g). That is why the drop in the absorption rate can be attributed to the shielding of the Compound 1 molecules by β-cyclodextrin, i.e. complex formation.

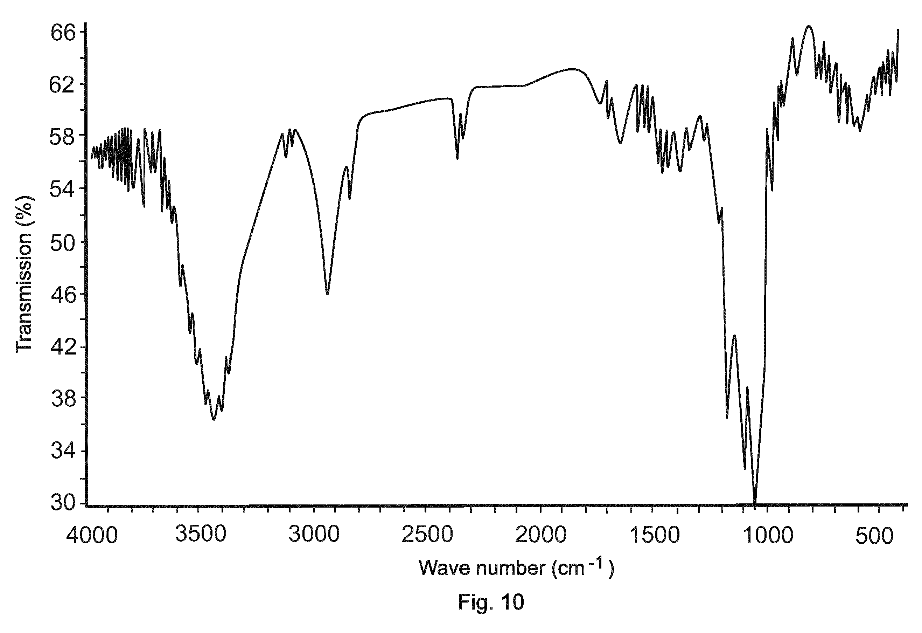

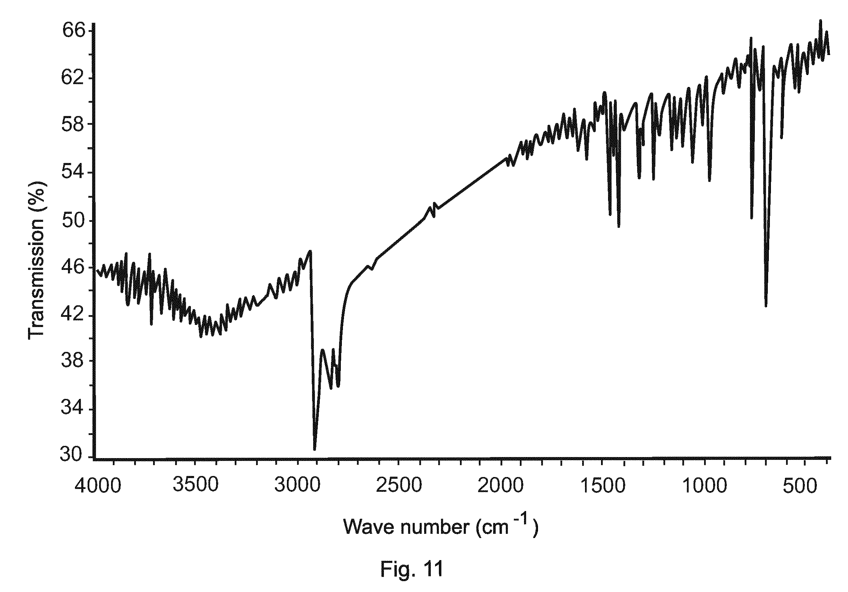

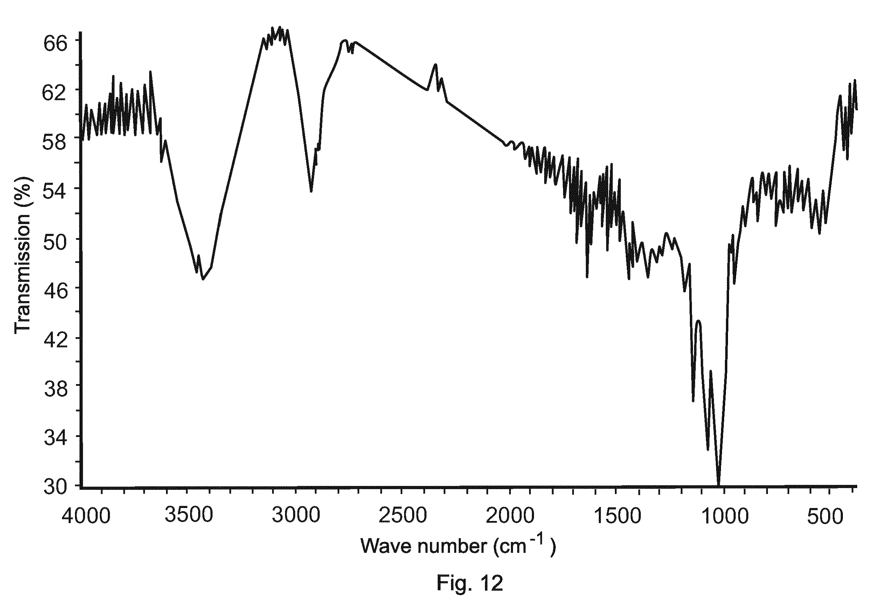

In addition, a comparative analysis of the IR spectra of β-cyclodextrin, compound 1 and compound 2 in the molar ratio of 1:3 (**Figs. 10****,** **11** **and** **12**, respectively) was carried out. The overlap of the signals of characteristic to Compound 1 groups, particularly of absorption band within the range 2800-2900 cm⁻¹, associated with the stretching vibrations of -CH₂ groups in saturated carbon atoms (strained cyclic structure), as well as the absorption band of 1400-1600 cm⁻¹ corresponding to the stretching vibrations of C-C of the benzene ring, indicates that the molecule of compound 1 is shielded in the cavity of the molecule of β-cyclodextrin. The overlap of the signals unambiguously confirm the formation of clathrate complexes.

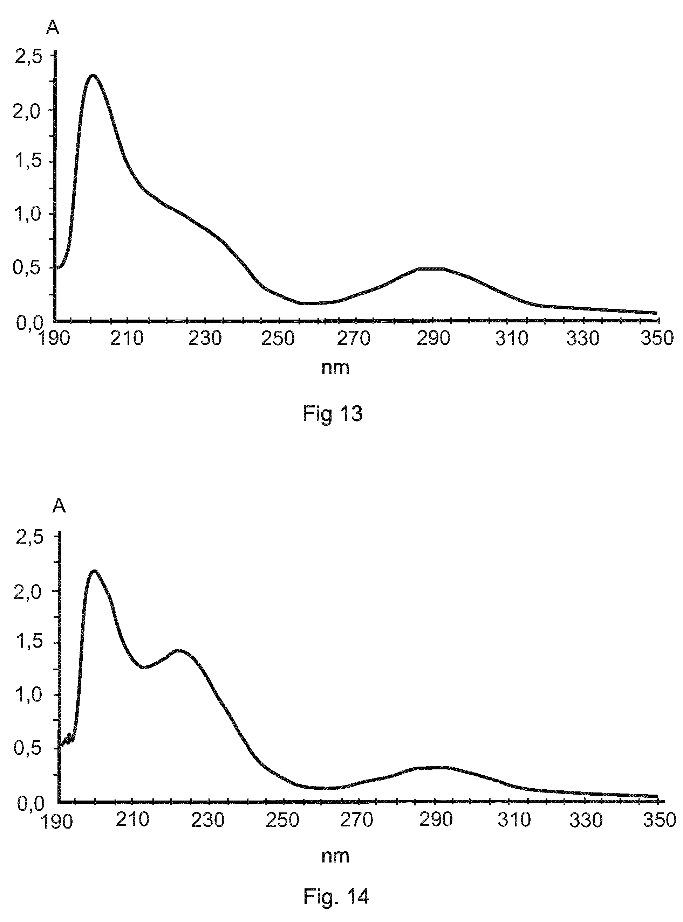

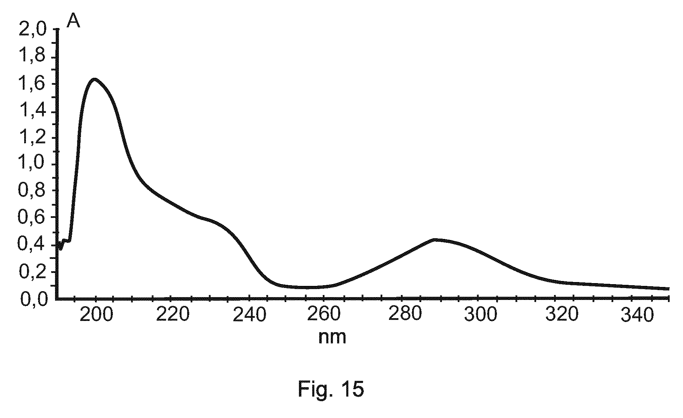

Furthermore, a comparative analysis of UV spectra of Compound 1 (**Fig. 6**), a complex of compound 1 and arabinogalactan (mass ratio 1:10 and 1:15) as well as pure arabinogalactan (**Figs. 13-15**, respectively) was performed. These spectra clearly reveal the lack of absorption in the region of 240-250 nm, which is characteristic for pure selenox. This confirms the fact that the formation of the complex selenox:arabinogalactan changes the spectral characteristics of the signals because of the shielding of selenox molecules by arabinogalactan molecules.

**TABLETS**

| | |
|---|---|
| Clathrate complex of Compound 1 and β-cyclodextrin at a potential molar ratio of 1:3 Possible additives: microcellulose, lactose, calcium stearate, starch. | 55 mg |

The tablets are prepared by mixing the ingredients in a *Bectochem* blender and press molding on a tablet machine *Rimec.*

**CAPSULES**

| | |
|---|---|
| Clathrate nanocomplex of Compound 1 and β-cyclodextrin in 1:5 molar ratio Possible additives: microcellulose, lactose, starch. | 90 mg |

Capsules are prepared by mixing the ingredients in a *Bectochem* blender and filling into gelatinous capsules 3VC.

**TABLETS**

| | |
|---|---|
| Clathrate complex of Compound 1 and arabinogalactan at a mass ratio of 1:10 Additives: microcellulose, lactose, calcium stearate, starch. | 60 mg |

The tablets are prepared by mixing the ingredients in a *Bectochem* blender and press molding on a tablet machine *Rimec.*

**CAPSULES**

| | |
|---|---|
| Clathrate nanocomplex of | 90 mg |

Compound 1:arabinogalactan in ratio of 1:15
Additives: microcellulose, lactose, starch.

Capsules are prepared by mixing the ingredients in a *Bectochem* blender and filling into gelatinous capsules 3VC.

**INJECTIONS**

| | |
|---|---|
| Clathrate nanocomplex of Compound 1 and β-cyclodextrin at a molar ratio of 1: 2 the solvent (water:ethanol). | 100 mg |

The solution is placed in pharmaceutically acceptable packing.

The study of radioprotective and cytoprotective properties of compound 1 and the complex compound 2 (ratio 1:3) by cloning method of hematopoietic stem cells in the spleens of irradiated mice.
1) Formation of spleen colonies by bone marrow cells of mice that received Compound 2 multiple times prior to irradiation at a dose of 1.5 Gy. Unlike compound 1, the complex form of compound 2 is water-soluble.
   Compound 2 was administered orally to mice-donors of bone marrow at doses of 5 mg/kg and 2 mg/kg in 0.2 ml physiological solution for 5 days. The dose of 5 mg/kg showed itself as the most effective single-application dose for the occurrence of an adaptive response to radiation. Control mice received 0.2 ml of physiological solution for 5 days. 7 days after the last injection, mice were irradiated (1.5 Gy at installation "Luch-1"). 15 minutes later the animals were sacrificed, a bone marrow cell suspension was prepared and injected to lethally irradiated recipient mice for determination of the colony-forming ability. The results are shown in **Table 1**. 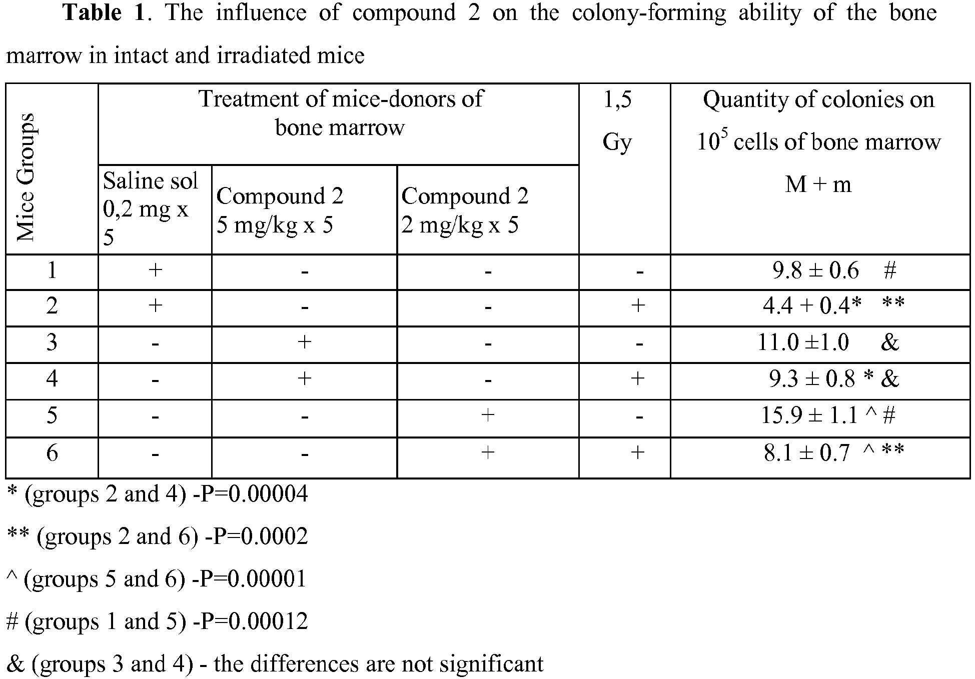
   The results presented in Table 1 show that five-time administration of the compound 2 at a dose of 5 mg/kg (effective single dose) by the 7^{th} day after the end of the course has not changed the number of colonies formed by the bone marrow cells of intact mice (Group 3). At the same time, the daily dose of 2 mg/kg contributed to the significant increase in yield of colonies compared to the control (Group 5). Both schemes of mice treatment prior to irradiation (1.5 Gy) were effective for generating the adaptive response of hematopoietic stem cells to irradiation. Moreover, the number of colonies formed in the group receiving 5 mg of compound 2 before the exposure was similar to the one at non-irradiated control animals.
2) Formation of spleen colonies by bone marrow cells of mice that received Compound 1, suspended in a starch gel, multiple times prior to irradiation at a dose of 1.5 Gy.

In the second series of experiments using the same experimental scheme and similar doses, the Compound 1 suspension was prepared in 2% starch gel. 90 female mice (CBA x C57B1/6) F1 were used in this study: 18 donors and 72 recipients. The data is presented in **Table 2**.

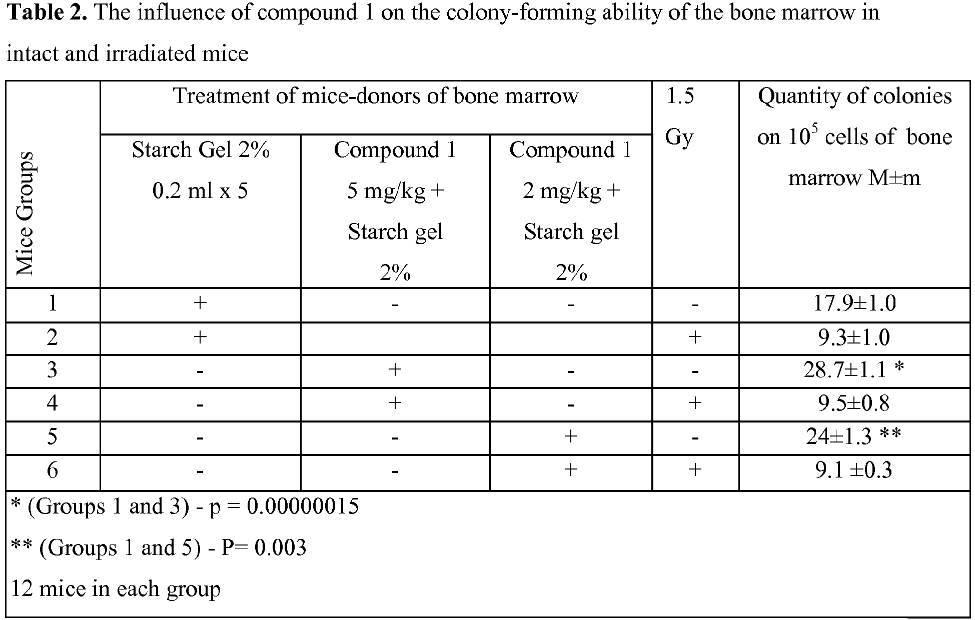

The Table 2 shows that treatment of mice with compound 1 (both doses tested) suspended in starch gel, promotes a statistically significant increase in the number of colonies formed by cells from the bone marrow in the spleens of irradiated recipient mice - Group 3 and 5. Radioprotection effect of Compound 1 suspended in the starch is absent.

A study on cytoprotective effect of the compound 1 was conducted. The data obtained is included **Table 3**.

**Table 3. Effect of compound 1 on the adaptive response of CFU-C-8 under single exposure of mice to Cisplatin**

| Group | Treatment of mice-donors of bone marrow | | | CFU-C-8 Quantity on 10⁵ bone marrow cells of donors |
|---|---|---|---|---|
| | Physiologic solution 0.2 ml | Compound 15 mg/kg | Cisplatin 5 mg/kg | |
| Control | + | - | - | 12.5 ± 0.6 |
| Compound 1 | + | + | - | 12.6 ± 0.6 |
| Cisplatin | - | - | + | 6.4 ± 0.6 |
| Compound 1 +Cisplatin | + | + | + | 9.6 ± 0.3* |
| * - p value in relation to group «Cisplatin» is equal to 0.004 | | | | |

According to the results shown in Table, compound 1 administered 7 days prior to a single injection of cisplatin to mice-donors of bone marrow provides distinct adaptive response of CFU-S-8 to this cytostatic agent.

Conclusions:
1. Mice daily treated with Compound 2 (5 mg/kg for 5 days) prior to irradiation, showed the development of an adaptive response of hematopoietic stem cells towards irradiation (1.5 Gy). The number of colonies formed by the bone marrow cells of the irradiated mice was comparable to the level of the intact control. Such treatment of control mice has not changed the number of exocolonies formed by the bone marrow cells.
2. Mice treated with Compound 2 (2 mg/kg for 5 days) before irradiation also showed the development of adaptive response of hematopoietic stem cells to radiation (1.5 Gy). The 5-day administration of 2 mg/kg of Compound 2 led to a significant increase for the number of exocolonies formed by the bone marrow cells.
3. Compound 1 suspended in starch gel, applied according to a protocol similar to that of compound 2, does not provide the radioprotective effect, although it significantly stimulates colonies output from intact bone marrow.
4. The results indicate that Compound 1 suspended in a starch gel affects the bone marrow of mice.
Fig. 1. The clathrate complex of β-cyclodextrin with 9-phenyl-sym-octahydroselenoxanthene in mass ratio of 1:10
Fig. 2. The clathrate complex of arabinogalactan with 9-phenyl-sym-octahydroselenoxanthene in mass ratio of 1:10
Fig. 3. The PMR-spectrum of β-cyclodextrin
Fig. 4. The PMR-spectrum of 9-phenyl-sym-octahydroselenoxanthene
Fig. 5. The PMR-spectrum of the clathrate complex of β-cyclodextrin with 9-phenyl-sym-octahydroselenoxanthene in mass ratio of 1:10
Fig. 6. The UV-spectrum of 9-phenyl-sym-octahydroselenoxanthene
Fig. 7. The UV-spectrum of the clathrate complex of β-cyclodextrin with 9-phenyl-sym-octahydroselenoxanthene in mass ratio of 1:10
Fig. 8. The UV-spectrum of the clathrate complex of β-cyclodextrin with 9-phenyl-sym-octahydroselenoxanthene in mass ratio of 1:17
Fig. 9. The UV-spectrum of β-cyclodextrin
Fig. 10. The IR-spectrum of β-cyclodextrin
Fig. 11. The IR-spectrum of 9-phenyl-sym-octahydroselenoxanthene
Fig. 12. The IR-spectrum of the clathrate complex of 9-phenyl-sym-octahydroselenoxanthene with β-cyclodextrin in mass ratio of 1:3
Fig. 13. The IR-spectrum of the clathrate complex of 9-phenyl-sym-octahydroselenoxanthene with arabinogalactan in mass ratio of 1:10
Fig. 14. The IR-spectrum of the clathrate complex of 9-phenyl-sym-octahydroselenoxanthene with arabinogalactan in mass ratio of 1:15
Fig. 15. The IR-spectrum of the arabinogalactan

## Claims

1. A clathrate complex of α-, β-, γ- or hydroxypropyl- β- cyclodextrin or arabinogalactan with 9-phenyl-sym-octahydroselenoxanthene of formula (I) 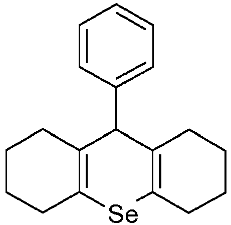 at a mass ratio of 9-phenyl-sym-octahydroselenoxanthene: cyclodextrin of from 1:3 to 1:30, or at a mass ratio of 9-phenyl-sym-octahydroselenoxanthene: arabinogalactan of from 1:10 to 1:20.

2. A clathrate complex of claim 1, wherein it is in crystalline form, in the form of nanoparticles with a size less than 100 nm.

3. The clathrate complex of claim 1, wherein the cyclodextrin is β-cyclodextrin.

4. The clathrate complex of claim 1 or of claim 3, wherein the mass ratio of 9-phenyl-sym-octahydroselenoxanthene: cyclodextrin is 1:10 or 1:17, in the form of nanoparticles with a size of approximately 24.3 nm.

5. The clathrate complex of claim 1 or of claim 2, wherein the mass ratio of 9-phenyl-sym-octahydroselenoxanthene:arabinogalactan is 1:10 or 1:15, in the form of nanoparticles with a size of approximately 32.5 nm.

6. The clathrate complex of claim 1 with a cytoradioprotective activity.

7. A liquid-phase method for preparing the clathrate complex of α-, β-, γ- or hydroxypropyl-β-cyclodextrin or arabinogalactan with 9-phenyl-sym-octahydroselenoxanthene of formula (I) according to claim 1, comprising the mixing of previously prepared aqueous solution of the appropriate cyclodextrin or arabinogalactan and the solution of 9-phenyl-sym-octahydroselenoxanthene in an organic water-miscible solvent, such as acetone, at a mass ratio of 9-phenyl-sym-octahydroselenoxanthene:cyclodextrin of from 1:3 to 1:30 and 9-phenyl-sym-octahydroselenoxanthene:arabinogalactan of from 1:10 to 1:20, under stirring and heating to a temperature up to 70°C, followed by stirring at the same temperature to obtain a homogeneous solution, isolating of the obtained clathrate complex, which is further milled, if necessary, to obtain a product in the form of nanoparticles with a size less than 100 nm.

8. The method of claim 7, comprising 9-phenyl-sym-octahydro-selenoxanthene:β-cyclodextrin in a mass ratio of 1:10 or 1:17, or 9-phenyl-sym-octahydroselenoxanthene:arabinogalactan in a mass ratio of 1:10 or 1:15.

9. A solid-phase method for preparing of clathrate complexes of α-, β-, γ- or hydroxypropyl-β-cyclodextrin or arabinogalactan with 9-phenyl-sym-octahydroselenoxanthene of claim 1, comprising the grinding of the corresponding cyclodextrin or arabinogalactan and 9-phenyl-sym-octahydroselenoxanthene at a mass ratio of 9-phenyl-sym-octahydroselenoxanthene: cyclodextrin of from 1:3 to 1:30, or 9-phenyl-sym-octahydroselenoxanthene: arabinogalactan of from 1:10 to 1: 20 in a ball mill at a speed of from 100 rpm to 1000 rpm in the range of from 10 to 60 min at a temperature ranging from 20 to 50°C to form the corresponding clathrate complex which, if necessary, is isolated in the form of nanoparticles with a size less than 100 nm.

10. The method of claim 9, comprising the complex of 9-phenyl-sym-octahydroselenoxanthene:β-cyclodextrin in a mass ratio of 1:10 or 1:17, and a 9-phenyl-sym-octahydroselenoxanthene:arabinogalactan in a mass ratio of 1:10 or 1:15.

11. A pharmaceutical composition with cytoradioprotective activity, comprising the effective quantity of clathrate complex of cyclodextrin or arabinogalactan with 9-phenyl-sym-octahydroselenoxanthene of claim 1 in the form of nanoparticles with a size less than 100 nm, and a pharmaceutically acceptable carrier and/or excipient.

12. The pharmaceutical composition of claim 11, comprising the clathrate complex of 9-phenyl-sym-octahydroselenoxanthene: β-cyclodextrin in a mass ratio of 1:10 or 1:17.

13. The pharmaceutical composition of claim 11, comprising the clathrate complex of 9-phenyl-sym-octahydroselenoxanthene:arabinogalactan in a mass ratio of 1:10 or 1:15.

14. A medicament with cytoradioprotective activity, in form of capsules, tablets or injections, placed in pharmaceutically acceptable packing, comprising the effective quantity of clathrate complex of β-cyclodextrin or arabinogalactan with 9-phenyl-sym-octahydroselenoxanthene of claim 1 or the pharmaceutical composition of claim 8.

15. The medicament of claim 14, comprising the clathrate complex of arabinogalactan with 9-phenyl-sym-octahydroselenoxanthene in mass ratio of 1:10 or 1:15.

16. The medicament of claim 14, comprising the clathrate complex of β-cyclodextrin with 9-phenyl-sym-octahydroselenoxanthene in mass ratio of 1:10 or 1:17.

## Patentansprüche

1. Clathratkomplex von α-, β-, γ- oder Hydroxypropyl- β- Cyclodextrin oder Arabinogalactan mit 9-Phenyl-sym-octahydroselenoxanthen der Formel (I) 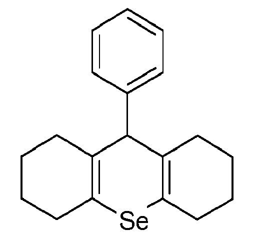 bei einem Massenverhältnis von 9-Phenyl-sym-octahydroxanthen: Cyclodextrin von 1: 3 bis 1:30, oder bei einem Massenverhältnis von 9-Phenyl-symoctahydroxanthene: Arabinogalactan von 1:10 bis 1:20.

2. Der Clathratkomplex nach Anspruch 1, wobei er in kristallinischer Form, in Form von Nanopartikeln mit einer Größe von weniger als 100 nm vorliegt.

3. Der Clathratkomplex nach Anspruch 1, wobei das Cyclodextrin als β-Cyclodextrin dargestellt ist.

4. Der Clathratkomplex nach Anspruch 1 oder 3, worin das Massenverhältnis von 9-Phenyl-sym-octahydroxanthen: Cyclodextrin 1:10 oder 1:17 ist, in Form von Nanopartikeln mit einer Größe von etwa 24,3 nm.

5. Der Clathratkomplex nach Anspruch 1 oder 2, worin das Massenverhältnis von 9-Phenyl-sym-octahydroxanthen: Arabinogalactan 1:10 oder 1:15 ist, in Form von Nanopartikeln mit einer Größe von etwa 32,5 nm.

6. Der Clathratkomplex nach Anspruch 1, der eine radioprotektive Aktivität hat.

7. Flüssigphasenverfahren zur Herstellung des Clathratkomplexes von α-, β-, γ- oder Hydroxypropyl-β-cyclodextrin oder von Arabinogalactan mit 9-Phenyl-sym-octahydroxanthen der Formel (I) nach Anspruch 1, umfassend die Vermischung von einer vorher vorbereiteten wässrigen Lösung von entsprechendem Ausgangscyclodextrin oder von Arabinogalactan mit einer Lösung non 9-Phenyl-sym-octahydroxanthen in einem organischen mit Wasser mischenden Lösungsmittel wie Aceton, bei einem Massenverhältnis von 9-Phenyl-sym-octahydroxanthen: Cyclodextrin von 1:3 bis 1:30 oder 9-Phenyl-sym-octahydroxanthen: Arabinogalactan von 1:10 bis 1:20, die Erhitzung der Lösung bis zu einer Temperatur von 70°C bei dem Vermischen und das weitere Vrnmischen bei der gleichen Temperatur, um eine homogene Lösung zu erhalten, die Isolierung vom erhaltenen Clathratkomplex, die falls erforderlich weiter zerkleinert wird, um ein Produkt in Form von Nanopartikeln mit einer Größe weniger als 100 nm zu erhalten.

8. Das Verfahren nach Anspruch 7, worin 9-Phenyl-sym-octahydroselenoxanthen: β-Cyclodextrin in einem Massenverhältnis 1:10 oder 1:17 oder 9-Phenyl-sym-octahydroselenoxanthen: Arabinogalactan in einem Massenverhältnis 1:10 oder 1:15 genutzt werden.

9. Festphasenverfahren zur Herstellung des Clathratkomplexes von α-, β-, γ- oder Hydroxypropyl-β-Cyclodextrin oder Arabinogalactan mit 9- Phenyl-sym-octahydroxanthen nach Anspruch 1, umfassend die Zermahlung von entsprechenden Cyclodextrin oder Arabinogalactan und 9-Phenyl-sym-octahydroxanthen bei einem Massenverhältnis von 9-Phenyl-sym-octahydroxanthen: Cyclodextrin von 1:3 bis 1:30, oder 9-Phenyl-sym-octahydroxanthen: Arabinogalactan von 1:10 bis 1: 20 in einer Kugelmühle bei einer Geschwindigkeit von 100 bis 1000 Umdrehungen pro Minute, im Bereich von 10 bis 60 Minuten bei einer Temperatur im Bereich von 20 bis 50°C, um den entsprechenden Clatratkomplex zu bilden, der notwendigerweise in Form von Nanopartikeln mit einer Größe weniger als 100 nm isoliert wird.

10. Das Verfahren nach Anspruch 9, worin 9-Phenyl-sym-octahydroxanthen: beta-Cyclodextrin in einem Massenverhältnis 1:10 und 1:17, und 9-Phenyl-sym-octahydroxanthen: Arabinogalactan in einem Massenverhältnis 1: 10 und 1:15 genutzt werden.

11. Eine pharmazeutische Zusammensetzung mit einer cytoradioprotectiven Aktivität, die eine wirksame Menge von Clathratkomplex von Cyclodextrin oder Arabinogalactan mit 9-Phenyl-sym-octahydroselenoxanthen nach Anspruch 1 in Form von Nanopartikeln mit einer Größe von weniger als 100 nm und pharmazeutisch akzeptable Träger und/oder Excipient enthält.

12. Die pharmazeutische Zusammensetzung nach Anspruch 11, die den Clathratkomplex von 9- Phenyl-sym-octahydroxanthen: beta-Cyclodextrin in einem Massenverhältnis 1:10 oder 1:17 enthält.

13. Die pharmazeutische Zusammensetzung nach Anspruch 11, die den Clathratkomplex von 9- Phenyl-sym-octahydroxanthen : Arabinogalactan in einem Massenverhältnis 1:10 oder 1:15 enthält.

14. Ein Arzneimittel mit einer cytoradioprotektiven Aktivität in Form von Kapseln, Tabletten oder Injektionen, das in einer verträglichen Dosierungsform ist und eine wirksame Menge von Clathratkomplex von beta-Cyclodextrin oder Arabinogalactan mit 9- Phenyl-sym-octahydroxanthen nach Anspruch 1 oder eine pharmazeutische Zusammensetzung nach Anspruch 11 enthält.

15. Das Arzneimittel nach Anspruch 14, das einen Clathratkomplex von 9-Phenyl-sym-octahydroxanthen mit Arabinogalactan in einem Massenverhältnis 1:10 oder 1:15 enthält.

16. Das Arzneimittel nach Anspruch 14, das einen Clathratkomplex von beta-Cyclodextrin mit 9-Phenyl-sym-octahydroxanthen in einem Massenverhältnis 1:10 oder 1:17 enthält.

## Revendications

1. Le complexe clathrate de α-, β -, *γ-* ou hydroxypropyl- β - cyclodextrine ou arabinogalactane avec 9-phényl-sym-octahydrosélénoxanthene de la formule (I) 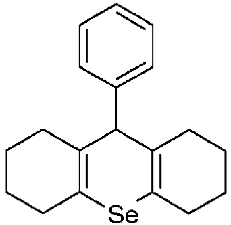 avec le rapport de masse de 9-phényl-sym-octahydrosélénoxanthene: cyclodextrine de 1:3 à 1:30, ou avec le rapport de masse de 9-phényl-sym-octahydrosélénoxanthene: arabinogalactane de 1:10 à 1:20.

2. Le complexe clathrate selon la revendication 1, où il a une forme crystalline, est en forme des nanoparticules moins de 100 nm.

3. Le complexe clathrate selon la revendication 1, où la cyclodextrine est β -cyclodextrine.

4. Le complexe clathrate selon la revendication 1 ou la revendication 3, où le rapport de masse de 9-phényl-sym-octahydrosélénoxanthene: cyclodextrine est 1:10 ou 1:17, en forme des nanoparticules environ 24.3 nm.

5. Le complexe clathrate selon la revendication 1 ou la revendication 2, où le rapport de masse de 9-phényl-sym-octahydrosélénoxanthene: arabinogalactane est 1:10 ou 1:15, en forme des nanoparticules environ 32,5 nm.

6. Le complexe clathrate selon la revendication 1 avec l'activité cito-radio-protective.

7. Une méthode de la phase liquide pour la préparation du complexe clathrate de α-, β -, *γ-*ou hydroxypropyl- β - cyclodextrine ou arabinogalactane avec 9-phényl-sym-octahydrosélénoxanthene de la formule (I) selon la revendication 1, qui comprend le mélange de la solution aqueuse préalablement préparé de la cyclodextrine approprié ou arabinogalactane et la solution de 9-phényl-sym-octahydrosélénoxanthene dans le solvant organique miscible à l'eau, comme l'acétone, avec le rapport de masse de 9-phényl-sym-octahydrosélénoxanthene: cyclodextrine de 1:3 à 1:30, ou avec le rapport de masse de 9-phényl-sym-octahydrosélénoxanthene: arabinogalactane de 1:10 à 1:20, en mélangeant et chaufferant à la température de 70°C, en maintenant le mélange et la temperature pour obtenir une solution homogène, en isolant du complexe clathrate obtenu, qui est broyé, si nécessaire, pour obtenir un produit en forme des nanoparticules moins de 100 nm.

8. Une méthode selon la revendication 7, qui comprend 9-phényl-sym-octahydrosélénoxanthene: cyclodextrine avec le rapport de masse de 1:10 ou 1:17, ou 9-phényl-sym-octahydrosélénoxanthene: arabinogalactane avec le rapport de masse de 1:10 ou 1:15.

9. Une méthode de la phase solide pour la préparation des complexes clathrates de α-, β -, γ-ou hydroxypropyl- β - cyclodextrine ou arabinogalactane avec 9-phényl-sym-octahydrosélénoxanthene selon la revendication 1, qui comprend le broyage de la cyclodextrine ou arabinogalactane et 9-phényl-sym-octahydrosélénoxanthene avec le rapport de masse de 9-phényl-sym-octahydrosélénoxanthene: cyclodextrine de 1:3 à 1:30, ou 9-phényl-sym-octahydrosélénoxanthene: arabinogalactane de 1:10 à 1:20 dans le broyeur à une vitesse de 100 rpm à 1000 rpm, pendant 10 à 60 minutes et à une temperature entre 20 et 50°C pour former le complexe clathrate correspondant qui, si c'est nécessaire, est isolé en forme des nanoparticules, de dimension moins de 100 nm.

10. Une méthode selon la revendication 9, qui comprend 9-phényl-sym-octahydrosélénoxanthene: β -cyclodextrine avec le rapport de masse de 1:10 ou 1:17, et 9-phényl-sym-octahydrosélénoxanthene: arabinogalactane avec le rapport de masse de 1:10 ou 1:15.

11. La composition pharmaceutique avec l'activité cito-radio-protective, qui comprend la quantité efficace du complexe clathrate de cyclodextrine ou arabinogalactane avec 9-phényl-sym-octahydrosélénoxanthene selon la revendication 1 en forme des nanoparticules moins de 100 nm, and le porteur acceptable pharmaceutiquement et/ ou l'excipient.

12. La composition pharmaceutique selon la revendication 11, qui comprend le complexe clathrate de 9-phényl-sym-octahydrosélénoxanthene: β -cyclodextrine avec le rapport de masse de 1:10 ou 1:17.

13. La composition pharmaceutique selon la revendication 11, qui comprend le complexe clathrate de 9-phényl-sym-octahydrosélénoxanthene: arabinogalactane avec le rapport de masse de 1:10 ou 1:15.

14. Le médicament avec l'activité cito-radio-protective, en forme de capsules, de comprimés ou d'injections placés dans un emballage pharmaceutique acceptable, qui comprend la quantité efficace du complexe clathrate de β -cyclodextrine ou arabinogalactane avec 9-phényl-sym-octahydrosélénoxanthene selon la revendication 1 ou la composition pharmaceutique selon la revendication 8.

15. Le medicament selon a revendication 14, qui comprend le complexe clathrate de l' arabinogalactane avec 9-phényl-sym-octahydrosélénoxanthene avec le rapport de masse de 1:10 ou 1:15.

16. Le medicament selon la revendication 14, qui comprend le complexe clathrate de β - cyclodextrine avec 9-phényl-sym-octahydrosélénoxanthene avec le rapport de masse de 1:10 ou 1:17.
